# EUROPEAN PATENT SPECIFICATION

(11) **EP 0 665 893 B1**
(45) Date of publication and mention of the grant of the patent: **10.07.2002**
(21) Application number: 93921955.6
(22) Date of filing: 14.10.1993
(51) Int. Cl.: C12Q 1/00, G01N 33/543, G01N 33/569

(54) **A METHOD AND AN APPARATUS FOR DETECTING CELLS**
VERFAHREN UND VORRICHTUNG ZUM NACHWEIS VON ZELLEN
PROCEDE ET APPAREIL POUR DETECTER DES CELLULES

(30) Priority: 23.10.1992 FI 924834
(43) Date of publication of application: 09.08.1995
(73) Proprietor: HAKALEHTO, Elias, 70110 Kuopio (FI)
(72) Inventor: HAKALEHTO, Elias, 70110 Kuopio (FI)
(86) International application number: FI9300418
(87) International publication number: WO9410336

(56) References cited:
- EP-A- 0 101 398
- EP-A- 0 299 601
- WO-A-88/08037
- WO-A-90/11522
- WO-A-91/05260
- GB-A- 2 252 260
- US-A- 4 303 752
- Dialog Information Services, File 154, Medline, Dialog Accession No. 07162459, Medline Accession No. 90069459, WIENECKA J. et al.: "Detection of Giardia Antigen in Stool Samples by a Semi-Quantitative Enzyme Immunoassay (EIA) Test.", Scand J Infect Dis, 1989, 21 (4) p 443-8.

## Description

### Field of the Invention

The present invention relates to a method and an apparatus for detecting cells, including viruses. The invention further relates to a kit suitable for use in the method of the invention. More specifically, the invention relates to a method for detecting cells, wherein the sample to be tested is filtered to separate the cells and the cells separated by the filter are treated to release therefrom the components employed in the cell detection reaction.

The detection of cells relates to applications in many scientific and technical fields. Such fields of application include for instance clinical and diagnostic testing, biotechnology and molecular biology as well as food and environmental analyses.

### Prior Art

Cells may be detected in a variety of ways both directly and indirectly. One indirect way is to determine certain cell components on the basis of their chemical, biological, immunological or physical properties, for instance.

Prior to the cell detection the sample must often be filtered, for example for the reason that the sample volume is so great and the cell content so low that the sensitivity of the methods employed in the detection reaction is not sufficient, or for the reason that it is desired to wash the cells on a filter prior to the actual determination. After the cells have been concentrated onto the filter and possibly washed, they are treated to release therefrom the components employed in the cell detection reaction, said components being determined on the filter (WO patent application 88/08037 and European Patent 101 398).

In cell detection methods of this kind, problems are presented by a number of impurities that impede the determination. Such impurities include e.g. the remaining cells and cell fragments and other solid particles present in the sample. Also the filter itself may provide an interfering background. Further, the method is laborious and the loss is great, particularly with samples having a low cell content.

In some other previously described methods (WO patent application WO 90/11522 and US patent 4,303,752) filtration can also be applied for concentrating the cells, but not for separating the non-cytoplasmic antigens from the remaining cells. The present method is thus offering a clear improvement when compared also with these methods, by e.g. adding specificity and removing background effects.

### Summary of the Invention

The object of the invention was to develop a method and an apparatus wherewith filtered cells can be reliably and conveniently detected. The method of the invention is characterized in that the components released from the cells are separated from the cells with the same filter that is employed for the filtering of the sample, and said components are determined from the filtrate to indicate the presence of cells in the original sample.

By detection of the cell components from the filtrate and not from the filter, impurities and the background provided by the filter as set forth above are avoided. Further, the possible pathogens in the sample may be inactivated, and thus they have no access to the filtrate to be analyzed. By separating the cell components that have been released from the cells and that are significant to the detection with the same filter that is employed for the concentration of the sample, labor costs and material costs as well as losses in the content of cells or cell components are minimized. Further, it is possible to simultaneously determine several cell components that may be derived from a variety of cell types.

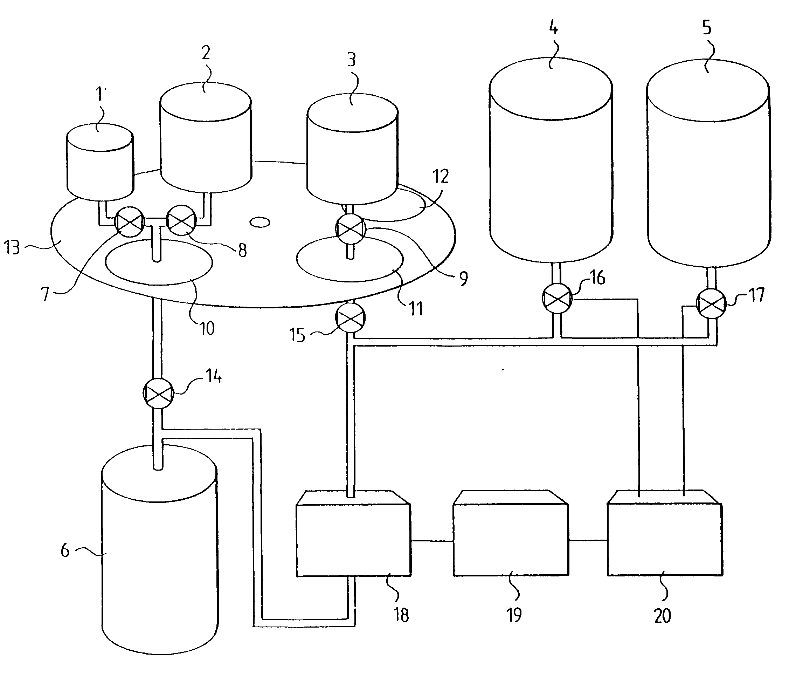

### Brief Description of the Drawing

The Drawing depicts an apparatus suitable for the method of the invention.

### Detailed Description of the Invention

The method of the invention may be applied to a wide variety of sample types. The method is well suited to samples having a large volume and a low cell content. The method is advantageously applied to liquid samples, but also other samples are possible, such as gas which may possibly first be extracted with a liquid. Samples that are initially in solid state may be dissolved or suspended in a liquid and possibly pre-filtered or the interfering substances removed by other means, by centrifugation for instance, prior to the detection. The method of the invention may be implemented for example in the food and beverage industry, in water, gas and air analyses, and in hygienic control in general as well as in clinical samples.

The sample to be tested is passed through a filter the pore size of which is selected on the basis of the size of the cells to be separated. In the present application, the term "cell" also encompasses viruses, even though viruses are not cells in the actual sense of the word but infection particles having an ability to penetrate a cell. Likewise, a cell component also denotes a virus component. The method of the invention is well suitable for the detection of bacteria and viruses, for instance. With bacteria, a filter having a pore size of 0.2 to 0.5 µm is generally used. With eucaryotic cells, a larger pore size is used, and with viruses, a smaller pore size - an ultrafilter - is used, respectively. For example suction or pressure may be employed in the filtration.

The filtration may be performed in conventional filtration apparatus wherein the filter is disposed on the bottom of a funnel, the funnel is placed on the opening of a suction bottle, and a vacuum is drawn into the suction bottle e.g. with a water pump. Alternatively, for instance an injection syringe may be employed, in which case the sample is drawn into the syringe, a filter is disposed at the end of the syringe, and the sample is pressed through the filter. It is naturally possible to employ other filtering systems as well. With viruses, it is practicable to perform ultrafiltration, wherein the sample is disposed on the filter of an ultrafiltration tube and is passed through the filter by centrifuging.

After the cells in the sample have been collected on the filter, it is often practicable to perform washing by passing a washing solution through the filter. Distilled water or a buffer, for instance, may be employed as the washing solution. The filtrates obtained thus far are discarded. After the possible washing, the cells are treated to release therefrom the cell components significant to the detection of the cells. The treatment may be carried out chemically or physically, and preferably in such a way that the cells or the majority of their texture remain on the filter. The chemical treatment may be enzymatic treatment, detergent treatment, extraction e.g. with an acid or base or some other suitable solvent, or flushing with water or a buffer solution, or an osmotic shock, for instance. The desired cell components may be physically detached from the cells for example by means of an ice press, by sonication, electroporation, shaking, or some other mechanical means.

The cell surface layer is preferably removed, said surface layer possibly in this connection also including extensions of cells, and specifically the bacterial flagella are detached and broken down and flagellin-containing structures or similar are determined. For example, the bacterial surface layer with its extensions may be removed by mild acid treatment. Thereby the flagella are detached and in that connection broken down into flagellin molecules. In the case of viruses, the antigens that are significant to the detection can be detached therefrom.

The detached components that are significant to the detection are flushed through the same filter that is employed for the filtration of the sample. The filtrate is collected, and the presence of cells in the original sample is detected from the flushing solution by means of reactions of the components that were passed through the filter on the basis of the chemical, biological, physical or immunological properties of the cell components. There are numerous methods of detecting cell components, including biochemical, immunological, and radiochemical analyses. The reagents necessary for the detection can also be passed through the filter, or they can be added directly to the filtrate.

The method of the invention can be employed both as a universal method for detecting cells and for the identification of a certain cell type. If the presence of cells in general is of interest, without any concern in the identification, the most practicable way is to define a component universally present in cells, such as LPS or structural parts thereof. Alternatively, the method may be employed to detect a certain cell type or strain that is of interest, in which case a cell component that is only present in the cell that is of interest is selected as the cell component to be determined. A very specific determination method is achieved by using immunological methods. It is possible to determine several cell components simultaneously from the same filtrate, and this is advantageous. In other words, it is possible to determine several cell types simultaneously by selecting the cell components to be determined in such a way that one component is only present in one cell type and another in another cell type, etc.

The method of the invention is very well suitable for determining a surface component in a cell, such as cell wall or cell membrane proteins, LPS, capsule polysaccharides, coat proteins (e.g. RS proteins, i.e. S-layer proteins), flagella or other extensions of cells or similar structures or their structural parts, etc. The released cell components are determined subsequent to separation from the main parts of the cells with a filter by conventional methods, such as a protein determination method or an immunological determination method. In principle, any protein detached from the cells can be employed to indicate the presence of cells in the original sample. For instance, gel electrophoresis, immunoblot analysis and immuno-PCR (immuno-polymerase chain reaction) technique (Sano, T., Smith, C. and Cantor, C., 1992, Science 258, pp. 120-122) are suitable for this purpose.

The method of the invention can also be applied to the detection of viruses both from impure samples and from cultures. The sample is placed on an ultrafilter through which it is passed by centrifugation, whereafter the filter is treated to detach the components employed in the detection, such as antigens, from the viruses that remain in the filter. At the same time, the advantage is achieved that pathogenic viruses can be inactivated. The detached antigens are passed through the same filter by recentrifugation, and the antigens are determined from the filtrate for instance by the immuno-PCR method, and hence very small antigen concentrations can be determined. In other words, the method of the invention is suitable for the concentration and purification of antigens and the pretreatment of samples to be tested by the immuno-PCR technique.

The scope of the invention also comprises an apparatus suitable for use in the method described above. The apparatus of the invention is characterized in that it comprises means for filtering the sample to be tested so as to separate the cells; means for treating the cells separated by the filter to release therefrom the components employed in the cell detection reaction; means for separating the released components from the cells, and means for determining said component from the filtrate to indicate the presence of cells in the original sample.

The means for filtering the sample to be tested so as to separate the cells can comprise for instance one or more vessels connected to a filter that is further connected to a waste container, for passing the sample and the possible washing liquid through the filter, and possibly means for passing the sample through the filter, such as a pump, a press or a centrifuge. The pore size of the filter is selected on the basis of the size of the cells to be separated.

The means for treating the cells separated by the filter to release therefrom the components employed in the cell detection reaction can comprise a vessel connected to the filter for passing the reagents that release cell components through the filter, or means for detaching cell components physically and means for passing a flushing solution through the filter.

The means for separating the released components from the cells consist of the same filter that is employed for filtering the sample, and possibly an intermediate part, such as transfer means enabling the use of the same filter. They may further comprise vessels that are connected to the filter for passing reagents through said filter on to said means for determining said cell component.

The means for determining the cell component comprise a measuring station, which may comprise an assay unit, a detector unit and an electronic unit for detecting the component released from the cells that is to be determined. The assay unit may comprise for instance different types of analysis systems, a light source etc. for the detection of components released from the cells on the basis of their chemical, biological, immunological or physical properties. The detector unit may comprise for instance light measuring means, radioactivity measuring means, etc. The measuring station normally further comprises an electronic unit for receiving the signal produced. The electronic unit is preferably connected to a data processing unit and a control unit. The measuring station is preferably also connected to vessels for conveying the running solutions and eluting solutions to the measuring station, the other end of which may be connected to a waste container. The measuring station is preferably such that several cell components may be determined therewith at the same time.

The apparatus of the invention usually comprises valves or other transfer means wherewith the reagents employed, the filter itself or the filtrate to be tested can be conveyed to the desired site.

The apparatus preferably comprises a sampling vat and a vat for the washing solution, which are connected to the filter, said filter being further connected to a waste container; transfer means for transferring the filter to another station whereto a third vat for the extracting solution is connected and which has a connection with the transferred filter, said filter being further connected to the measuring station, the data processing unit and the control unit; and vessels for the running solution and the eluting solution, said vessels also being connected to the measuring station.

With the apparatus of the invention, the method of the invention can be largely automated. The invention also relates to the use of the apparatus in the method of the invention. Preferably the apparatus is employed to detect several cell components simultaneously from the same sample.

The drawing shows an example of the apparatus of the invention. It is evident that the illustrated embodiment is not the only possible within the scope of the invention.

The drawing shows a sampling vat 1 that is connected via a valve 7 to a filter 10 and via a valve 14 to a waste container 6 for passing the sample through the filter into the waste container. Further, another vat 2 is connected to the filter via a valve 8 for passing a washing liquid through the filter. The apparatus further comprises transfer means 13 for transferring the filter to stations 11 and 12. A vat 3 is connected to station 11 via a valve 9 for supplying an extracting solution to the filter. The filter at station 11 is connected to a measuring station 18 via a valve 15 for supplying the extracting solution to the measuring station whereto a vat 4 is connected via a filter 16 for supplying a running solution thereto. The measuring station is connected to a data processing unit 19 for the interpretation and output of the signal. The apparatus also comprises a control unit 20 for controlling the operation of the valves and other mechanical actuators of the apparatus and for supplying a current. Further, a vat 5 is connected to the measuring station via a valve 17 for supplying the eluting solution to said measuring station for regeneration. Station 12 is a filter exchange station.

The apparatus described above is used as follows: The sample to be tested is supplied to the sampling vat 1 wherefrom it is passed by means of pressure via valve 7 through filter 10 and valve 14 into the waste container 6. The cells remaining on the filter are washed with a washing solution supplied through valve 8 from vat 2. Hereafter the filter is transferred with the transfer means 13 to station 11 at which the cells remaining on the filter are extracted with a solution from vat 3 regulated by valve 9. (Instead of transferring the filter, it is naturally possible to convey the extracting solution to a stationary filter wherefrom the filtrate is conveyed to the measuring station for instance via appropriate valves.) The extract is driven via valve 15 to the measuring station 18 through which it is passed by means of the running solution from vat 4, supplied through filter 16. The signal derived from the measuring station is interpreted by means of the data processing unit 19. The data processing unit outputs the interpreted signal and assesses the presence and amount of cell components to be determined. The control unit 20 controls the operation of the valves and other mechanical actuators in the apparatus and supplies current to the system. After the measuring, the measuring station is regenerated with eluting solution from vat 5, supplied via valve 17. At station 12, the spent filter is replaced by a new filter.

The invention further relates to a kit comprising the means employed in the method. The kit is characterized in that it comprises means for filtering the sample to be tested to separate the cells; means for treating the cells separated by the filter to release therefrom the components employed in the cell detection reaction; means for separating the released components from the cells, and means for determining said component from the filtrate to indicate the presence of cells in the original sample.

The means for filtering the sample to be tested to separate the cells preferably comprise filters and appertaining vessels suitable for filtering, and possibly the necessary washing solutions. The means for treating the cells preferably comprise reagents for releasing the components employed in the cell detection reaction, and the means wherewith the components are separated from the cells consist of the same filter that is employed for the filtration of the sample. The means for determining the component from the filtrate comprise the reagents employed in the determination. The apparatus employed in the determination is normally not included in the kit.

The filters in the kit of the invention are selected on the basis of the size of the cells to be separated. The vessels to be connected to the filter that are suitable for filtering may be funnels connected to a suction bottle, injection syringes, or ultrafiltration tubes. The reagent to be employed for releasing the cell components may be for example an enzyme, a detergent, an acid or a base solution or some other solvent, water or a buffer. The reagents needed for the detection of components released from the cells may be any reagents necessary in a conventional reaction for the detection of cell components, such as reagents employed in protein determination or immunoassays.

The kit of the invention preferably contains sample and reagent vessels, filters, reagents for releasing cell components employed in the detection reaction, and reagents for determining the released components.

The invention will be explained in more detail by means of the following non-limiting examples.

### Example 1

Bacterial cultures of 1.5 ml containing the Pectinatus frisingiensis strain VTT-E-82165 (Hakalehto, E. and Finne, J., 1990, FEMS Microbiology Letters 67, pp. 307-312) were drawn into a 2 ml injection syringe and pressed through a filter disposed at the end of the syringe, the filter having a pore size of 0.45 µm (Millipore Millex HA). The cells were flushed with distilled water. The filtrates obtained were discarded. About 0.2 ml of 0.05 M HCl was then passed through the same filter using another syringe, and the filtrate was collected in an Eppendorff tube and neutralized with NaOH.

Thereafter a sample buffer employed in SDS polyacrylamide gel electrophoresis (SDS-PAGE) was added to the filtrates, and SDS-PAGE was performed on them, as described in Laemmli, U.K., 1970, Nature (London) 227, pp. 680-685, using a 8% gel and commercial molecular weight standards (Pharmacia). Some of the samples were boiled prior to the run. The gels were stained with the protein dye Coomassie Brilliant Blue (CBB) and/or immunoblotting was performed using an antibody raised in a rabbit immunized with cells of said bacterial strain (Hakalehto, E. and Finne, J., 1990, FEMS Microbiology Letters 67, pp. 307-312). Both in direct staining and in immunoblot analysis, a distinct band was found at about 63 kDa, indicating the presence of flagellin protein of the size of about 63 kDa derived from the Pectinatus frisingiensis strain. This again is an indication of the presence of the Pectinatus frisingiensis strain in the original sample.

### Example 2

A sample containing the bacterium Bacillus polymyxa (ATCC 842) was treated as in Example 1, and SDS-PAGE was run on the collected filtrate using a 10% gel which was subsequently stained with CBB. A distinct band was to be seen at 120-130 kDa, corresponding to the RS (Regularly Structured) protein present in the coat of these bacteria, said RS protein having a molecular weight of about 120-130 kDa.

### Example 3

A sample containing the bacterium Enterobacter aerogenes (ATCC 13048) was treated as in Example 1. SDS-PAGE analysis was performed on the collected filtrate with a 8% gel, to give a distinct protein band slightly below the 63 kDa protein of the Pectinatus strain described in Example 1. After purification and sequential analysis, it was found that the protein band corresponds to the flagellin of the Enterobacter strain.

### Example 4

A sample containing all of the three bacteria mentioned in Examples 1 to 3, i.e. P. frisingiensis VTT-E-82165, B. polymyxa ATCC 842 and E. aerogenes ATCC 13048, was treated as in Example 1, and SDS-PAGE was run on the collected filtrate using a 8% gel. Distinct bands were obtained at the locus of the bands obtained in Examples 1 to 3, which indicated the presence of all three bacteria in the original sample.

### Example 5

400 ml of a sample containing 0.2 ml of a culture of the bacterium E. aerogenes (ATCC 13048) was filtrated using a Millipore HA filter with a pore size of 0.45 µm and a diameter of 25 mm. The filtered culture was washed with distilled water or a buffer. The filtrates were discarded. Thereafter 200 µl of 0.05 M HCl was added to the filter, and the filtrate was drawn into an Eppendorff tube. NaOH was added for neutralizing purposes, and finally flushing with distilled water was performed employing suction. Thereafter SDS-PAGE was run on the sample collected in the Eppendorff tube, as in Example 1, using a 12% gel.

Silver staining was performed on the gel as follows: The gel was first shaken in 50% methanol, whereafter it was transferred to a silver solution prepared by adding dropwise 0.8 g of AgNO₃ in 4 ml of distilled water to a solution into which 0.36% of NaOH (21 ml) and ammonia (1.4 ml) had been mixed. Subsequent to the silver solution, the gel was washed with distilled water and transferred to a solution containing 1.25 ml of 1% citric acid and 0.125 ml of 30% formaldehyde in an aqueous solution (250 ml). After the stained protein bands had appeared, the gel was washed with water and transferred to a solution containing 45% of methanol and 1% of acetic acid. The most distinct protein bands were obtained at about 21 kDa and 29 kDa. Also other components detached from cells were detected, but the bands were weaker.

### Example 6

A Coxsackie B 6 virus, belonging to the Picorna viruses, obtained from a patient sample was cultivated in a monkey kidney cell culture, whereafter the viruses were released from the cells by freezing and thawing the culture several times. 500 µl of this virus suspension was mixed in 500 µl of deionized water and laid on the filter of an ultrafiltration tube (Microsep 10K, Filtron, USA) and centrifuged for half an hour, 4000 - 5000 rpm. No washing had to be performed. 100 µl of 0.05 M HCl was added to the filter and was allowed to react for one hour at 37°C, whereafter centrifugation was carried out as above. 100 µl of 0.05 M NaOH was added to the filter and recentrifuged as above. SDS-PAGE was run on the filtrate (in 10% gel) as in Example 1. Prior to the run, the samples were boiled for 5 minutes in SDS-PAGE sample buffer. The gel was stained by silver staining as in Example 5, or an immunoblot analysis was performed as in Example 1. An anti-Coxsackie B 6 antibody raised in a monkey (diluted 1:50) was employed as the first antibody, and 1:1000 diluted anti-human antibody labeled with alkaline phosphatase (Dako, Denmark) was employed as the second antibody, of the immunoblot. The anti-human antibody cross-reacted with the monkey antibody.

The silver-stained SDS gel had several bands that were partly different from the bands obtained with boiled whole viruses, respectively. In the immunoblot method, four bands were highlighted in the range 25 - 32 kDa. According to the literature, the most significant structural proteins of Picorna viruses are normally in the range 9-35 kDa (Andrews, C., Pereira, H.G. and Wildy, P., 1978. Viruses of Vertebrates. Bailliere Tindall, London).

## Claims

1. A method for detecting cells, comprising
a) filtering a sample to be tested so as to separate the cells,
b) treating the cells on the filter to release by chemical or physical means therefrom the surface components employed in the cell detection reaction, which surface components mean macromolecules, their complexes, or their structural parts deriving from the cell appendages such as flagella or other extensions of the cells, or from capsules, RS-layer, or LPS, or outer membrane, or other corresponding structure,
c) separating from the main parts of the cells the said surface components released therefrom with the same filter that is employed for the filtering of the sample, without having in the filtrate impurities from the remaining parts of the cells or from the filter, which impurities would impede the detection reaction, and
d) determining said surface components from the filtrate to indicate the presence of cells in the original sample.

2. A method as claimed in claim 1, which is applied to the detection of bacteria.

3. A method as claimed in one of the claims 1 or 2, in which surface layer of the cells, including the extensions of the cells, is removed.

4. A method as claimed in one of the claims 2 or 3, in which the flagella of the bacteria are detached and broken and flagellin-containing structures or similar structures are determined.

5. A method as claimed in one of the claims 2 or 3, in which RS-protein of the bacteria is determined.

6. A method as claimed in claim 1, in which a surface component present in extensions, coat or cell wall of the bacteria is determined.

7. A method as claimed in claim 1, in which the said surface components are released and determined by an immuno-assay.

8. A method as claimed in claim 1, in which several of the said surface components are determined from the filtrate simultaneously to detect the presence of several cell types in the same sample.

9. A method as claimed in claim 1, in which an antigen to be determined by the immuno-PCR method is released from the cell surface.

10. A method as claimed in any of the claims 1-9, in which the reagents necessary for the detection of the surface components can be passed through the same filter that is employed for the filtering of the sample.

11. Use of an apparatus comprising
a) means for filtering the sample to be tested so as to separate the cells;
b) means for treating the cells separated by the filter to release therefrom the said surface components employed in the cell detection reaction;
c) means for separating the said surface components released from the cells, said means consisting of the same filter that is employed for filtering the sample; and
d) means for determining said surface components from the filtrate to indicate the presence of cells in the original sample; in the method of claim 1 for the detection of cells.

12. The use according to claim 11, in which the apparatus is used to determine several of the said surface components simultaneously.

## Patentansprüche

1. Ein Verfahren zur Entdeckung von Zellen, bestehend aus
a) Filtern einer zu testenden Probe für die Zerteilung der Zellen,
b) Behandlung der Zellen auf dem Filter zur Lösung der Oberflächenkomponenten mit chemischen oder physischen Mitteln, die zu der Reaktion der Zellenentdeckung beitragen, welche Oberflächenkomponenten Makromoleküle, deren Kombinationen, oder deren strukturelle Teile, die sich von den Zellenanhängseln ableiten, wie Gebinde oder sonstige Streckung der Zellen, oder Kapsel, RS-Schicht oder LPS, oder eine Außenmembran oder sonstige entsprechende Struktur bedeuten,
c) Zerteilung von den Hauptteilen der Zellen die gesagten Oberflächenkomponenten, die mit dem gleichen Filter davon gelöst worden sind, welcher für die Filterung der Probe eingesetzt wird, ohne dass im Filtrat Unreinheiten von den zurückgebliebenen Teilen der Zellen oder vom Filter vorhanden sind, welche Unreinheiten die Entdeckungsreaktion verhindern würden, und
d) Feststellen der gesagten Oberflächenkomponenten aus dem Filtrat um die Anwesenheit der Zellen in der Originalprobe anzuzeigen.

2. Ein Verfahren nach Anspruch 1, welche für die Entdeckung von Bakterien verwendet wird.

3. Ein Verfahren nach einem der Ansprüche 1 oder 2, wobei die Oberflächenschicht der Zellen, einschließlich der Streckung der Zellen, entfernt wird.

4. Ein Verfahren nach einem der Ansprüche 2 oder 3, wobei die Bakteriengebinde gelöst und gebrochen werden und die gebindeenthaltenden Strukturen oder ähnliche Strukturen definiert werden.

5. Ein Verfahren nach einem der Ansprüche 2 oder 3, wobei RS-Protein der Bakterien definiert wird.

6. Ein Verfahren nach Anspruch 1, wobei eine in den Streckungen, am Mantel oder an der Zellenwand der Bakterien vorhandene Oberflächenkomponente der Bakterien definiert wird.

7. Ein Verfahren nach Anspruch 1, wobei die gesagten Oberflächenkomponenten gelöst und mit einer Antiprobe definiert werden.

8. Ein Verfahren nach Anspruch 1, wobei mehrere der gesagten Oberflächenkomponenten aus dem Filtrat gleichzeitig definiert werden, um die Anwesenheit verschiedener Zellentypen in der gleichen Probe zu entdecken.

9. Ein Verfahren nach Anspruch 1, wobei ein mit dem Anti-PCR-Verfahren definiertes Antigen von der Zellenoberfläche gelöst wird.

10. Ein Verfahren nach einem der Ansprüche 1-9, wobei die für die Entdeckung der Oberflächenkomponenten erforderlichen Reagenzien durch den gleichen Filter durchgedrückt werden können, welcher für die Filterung der Probe eingesetzt wird.

11. Verwendung eines Apparats, bestehend aus
a) Mitteln für die Filterung der zu testenden Probe zur Zerteilung der Zellen;
b) Mitteln für die Behandlung der durch den Filter zerteilten Zellen, um daraus die gesagten Oberflächenkomponenten zu lösen, die bei der Reaktion der Entdeckung der Zellen verwendet werden;
c) Mitteln für die Zerteilung der von den Zellen gelösten erwähnten Oberflächenkomponenten, die erwähnten Mittel bestehend aus dem gleichen Filter, der für die Filterung der Probe verwendet wird; und
d) Mitteln für die Definierung der erwähnten Oberflächenkomponenten aus dem Filtrat, um die Anwesenheit der Zellen in der Originalprobe anzuzeigen; im Verfahren nach Anspruch 1 für die Entdeckung von Zellen.

12. Die Verwendung nach Anspruch 11, wobei der Apparat für die Definierung verschiedener der erwähnten Oberflächenkomponenten gleichzeitig verwendet wird.

## Revendications

1. Un procédé de détection de cellules, comprenant
a) le filtrage d'un échantillon à analyser, afin de séparer les cellules,
b) le traitement des cellules sur le filtre pour en libérer, par un moyen chimique ou physique, les composants de surface utilisés dans la réaction de détection de cellules, lesquels composants de surface signifient macromolécules, leurs complexes ou leurs éléments structurels dérivant des appendices cellulaires tels que flagelles ou autres prolongations des cellules, ou des capsules, de la couche RS ou du LPS, de la membrane extérieure ou d'une autre structure correspondante,
c) la séparation desdits composants de surface des éléments principaux des cellules, dont ils sont libérés, à l'aide du même filtre qui est utilisé pour le filtrage de l'échantillon, sans avoir, dans le filtrage, des impuretés des parties restantes des cellules ou du filtre, lesquelles impuretés gênerainent la réaction de détection, et
d) la détermination desdits composants de surface dans le filtrat, pour indiquer la présence des cellules dans l'échantillon d'origine.

2. Un procédé suivant la revendication 1, qui est appliqué à la détection de bactéries.

3. Un procédé suivant l'une des revendications 1 ou 2, dans lequel la couche de surface des cellules, les prolongations des cellules incluses, est enlevée.

4. Un procédé suivant l'une des revendications 2 ou 3, dans lequel les flagelles des bactéries sont détachés et cassés et les structures contenant de la flagelline ou les structures similaires sont déterminées.

5. Un procédé suivant l'une des revendications 2 ou 3, dans lequel la protéine RS des bactéries est déterminée.

6. Un procédé suivant la revendication 1, dans lequel un composant de surface présent dans les prolongations, l'enveloppe ou la paroi cellulaire des bactéries est déterminé.

7. Un procédé suivant la revendication 1, dans lequel lesdits composants de surface sont libérés et déterminés à l'aide d'une immuno-analyse.

8. Un procédé suivant la revendication 1, dans lequel plusieurs composants de surface sont déterminés du filtrat simultanément afin de détecter la présence de plusieurs types de cellules dans le même échantillon.

9. Un procédé suivant la revendication 1, dans lequel un antigène à déterminer avec la méthode immuno-PCR est libéré de la surface cellulaire.

10. Un procédé suivant l'une des revendications de 1 à 9, dans lequel les réactifs nécessaires pour la détection des composants de surface peuvent être passés à travers le même filtre qui est utilisé pour le filtrage de l'échantillon.

11. L'utilisation d'un appareillage comprenant
a) un moyen pour le filtrage de l'échantillon à tester afin de séparer les cellules;
b) un moyen pour le traitement des cellules séparées avec le filtre afin d'en libérer les dits composants de surface utilisés dans la réaction de détection de cellules;
c) un moyen pour la séparation desdits composants de surface libérés des cellules, ledit moyen étant composé du même filtre qui est utilisé pour le filtrage de l'échantillon; et
d) un moyen pour la détermination, du filtrat, desdits composants de surface, afin d'indiquer la présence des cellules dans l'échantillon d'origine; dans le procédé de la revendication 1 pour la détection de cellules.

12. L'utilisation suivant la revendication 11, dans laquelle l'appareillage est employé pour la détermination de plusieurs composants de surface simultanément.
